# EUROPEAN PATENT APPLICATION

(11) **EP 4 393 904 A1**
(43) Date of publication of application: **03.07.2024**
(21) Application number: 21958182.4
(22) Date of filing: 29.11.2021
(51) Int. Cl.: C07C 15/04, C07C 15/06, C07C 15/08, B01J 29/06, B01J 29/40, B01J 37/08, B01J 37/10

(54) **METHOD FOR COUPLING AND CONVERTING NAPHTHA AND CARBON DIOXIDE TO PRODUCE BENZENE, TOLUENE AND P-XYLENE**

(30) Priority: 24.09.2021 CN 202111124097
(71) Applicant: Dalian Institute Of Chemical Physics, Chinese Academy Of Sciences, Liaoning 116023 (CN)
(72) Inventor: LIU, Zhongmin, Dalian, Liaoning 116023 (CN); YU, Zhengxi, Dalian, Liaoning 116023 (CN); YANG, Yue, Dalian, Liaoning 116023 (CN)
(74) Representative: Bayramoglu et al.
(86) International application number: PCT/CN2021/133888
(87) International publication number: WO 2023/045070

(57) **Abstract**

Disclosed are a method for preparing a modified molecular sieve catalyst and a method for producing benzene, toluene and p-xylene by coupling conversion of naphtha and CO₂. The method for preparing a modified molecular sieve catalyst includes subjecting a molecular sieve to metal modification by using a high temperature hydrothermal method, which includes the following steps: (1) preparing a soluble metal salt aqueous solution; (2) placing a zeolite molecular sieve to be metal-modified in the soluble metal salt aqueous solution, and impregnating the same at a temperature of 60-100°C; and (3) draining the molecular sieve obtained in step (2), followed by drying and calcination. The method for producing benzene, toluene and p-xylene by coupling conversion of naphtha and CO₂ includes: (a) preparing a modified molecular sieve catalyst; and (b) enabling a raw material containing naphtha and CO₂ to contact with the modified molecular sieve catalyst in a reactor for a reaction to produce benzene, toluene and p-xylene; and the method overcomes the defects of limited hydrogen resources and high costs of existing technologies.

## Description

### FIELD OF TECHNOLOGY

The present invention belongs to the field of petrochemical industry, and specifically relates to a method for producing benzene, toluene and p-xylene by coupling conversion of naphtha and CO₂, in particular to a method for producing benzene, toluene and p-xylene by coupling conversion of naphtha and CO₂ under catalysis of a modified zeolite molecular sieve catalyst.

### BACKGROUND

With the development of industry, the amount of carbon dioxide (CO₂) in atmosphere is constantly increasing, leading to a more and more serious greenhouse effect. In response to the international concept of "carbon neutrality", recycling of CO₂ has become the research focus of scientific research personnel.

In addition, benzene, toluene and xylene (hereinafter referred to as BTPX) are basic chemicals, which have large market consumption and need to be imported in large quantities in China. At present, a technical route of producing BTPX mainly involves catalytic reforming of naphtha. However, the technical route still cannot meet demands in China.

Although researchers have found that hydrogenation of carbon dioxide under the action of hydrogen can be used for producing BTPX, this route is used for producing liquid hydrocarbons or BTPX by hydrogenation of carbon dioxide, technical indicators are difficult to achieve, and sources of hydrogen have become a key problem limiting industrial application.

### SUMMARY

In order to solve the above technical problems, the inventor of the present application has creatively found that coupling of naphtha as a raw material and CO₂ as a raw material to produce benzene, toluene and p-xylene is a new technical route of producing aromatic benzene, toluene and p-xylene by means of CO₂, which provides a new way for production of benzene, toluene and p-xylene and large-scale utilization of CO₂.

On the one hand, the present application provides a method for preparing a modified molecular sieve catalyst used for catalyzing coupling conversion of naphtha and CO₂ to produce benzene, toluene and p-xylene, where the method includes subjecting a molecular sieve to metal modification by using a high temperature hydrothermal method, including the following steps:
(1) preparing a soluble metal salt aqueous solution;
(2) placing a zeolite molecular sieve to be metal-modified in the soluble metal salt aqueous solution, and impregnating the same at a temperature of 60-100°C; and
(3) draining the molecular sieve obtained in step (2), followed by drying and calcination to obtain the modified molecular sieve catalyst.

Optionally, the solid-liquid ratio of the zeolite molecular sieve to be metal-modified to the soluble metal salt aqueous solution is 1/10 to 1/1, and the mass concentration of a metal salt in the soluble metal salt aqueous solution is 10%-30%; the impregnating time is 2-10 hours; the step of drying is carried out in an air atmosphere at 100-150°C; and the step of calcination is carried out in an air atmosphere at 500-700°C.

Optionally, in step (1), the soluble metal salt aqueous solution is heated to any value or a range value determined by any two values of the following temperatures: 60°C, 70°C, 80°C, 90°C and 100°C.

Optionally, in step (2), the impregnating time is any value or a range value determined by any two values of the following times: 2 hours, 4 hours, 6 hours, 8 hours and 10 hours.

Optionally, a metal used for the metal modification is selected from at least one of La, Zn, Ga, Fe, Mo and Cr metals.

Optionally, the metal modification includes modification with a duplex metal of Zn and Ga.

Optionally, the modified zeolite molecular sieve catalyst consists of a modified HZSM-5 zeolite molecular sieve.

Optionally, the modified zeolite molecular sieve catalyst includes a modified HZSM-5 zeolite molecular sieve and a binder.

Optionally, the method further includes subjecting the molecular sieve to silanization modification after the metal modification.

Optionally, the silanization modification is carried out by using an in situ chemical vapor deposition method, which includes the following steps:
(4) placing the metal-modified zeolite molecular sieve in a reactor;
(5) introducing a material A containing a silanization reagent into the reactor at one time, where the introduced amount of the silanization reagent is 0.2-0.3 g/g solid, and the silanization reagent is gaseous in the reactor; and
(6) stopping the introduction of the material A into the reactor, raising the temperature of the reactor to 400°C or above, and introducing air for calcination.

Optionally, in step (6), the temperature of the reactor is raised to 400-550°C, and the air is introduced for calcination.

Specifically, the silanization modification is carried out by using an in situ chemical vapor deposition method, which includes the following steps:
(4-1) placing the metal-modified zeolite molecular sieve in a reactor, carrying out activation treatment with nitrogen under high temperature conditions (such as 450-550°C), and then carrying out cooling to a silanization modification temperature in a nitrogen atmosphere (the temperature ensures that a silanization reagent is in a gaseous state in the reactor);
(5-1) introducing a material A containing all the silanization reagent into the reactor at one time, and continuing the introduction for predetermined time, where the material A is a silanization reagent with nitrogen as a carrier gas; and
(6-1) stopping the introduction of the material A into the reactor, raising the temperature of the reactor to 400°C or above (such as 550°C), and introducing air for calcination to complete the silanization modification of the zeolite molecular sieve.

Optionally, the silanization modification is carried out by using an in situ vapor atomic layer deposition method, which includes the following steps:
(4') placing the metal-modified zeolite molecular sieve in a reactor;
(5') introducing a material A containing a silanization reagent into the reactor at n times, where the introduced amount of the silanization reagent each time is 0.03-0.06 g/g solid, the silanization reagent is gaseous in the reactor, and the n is in a value range of 3-6; and
(6') stopping the introduction of the material A into the reactor, raising the temperature of the reactor to 400°C or above, and introducing air for calcination.

Specifically, the silanization modification is carried out by using an in situ vapor atomic layer deposition method, which includes the following steps:
(4'-1) placing the metal-modified zeolite molecular sieve in a reactor;
(5'-1) introducing nitrogen as a carrier gas into a saturated flask filled with a silanization agent, introducing the carrier gas carrying the silanization agent into the reactor continuously for predetermined time, carrying out activation treatment with nitrogen under high temperature conditions (such as 450-550°C), and then carrying out cooling to a silanization modification temperature in a nitrogen atmosphere (the temperature ensures that the silanization reagent is in a gaseous state in the reactor);
(6'-1) stopping the introduction of the silanization reagent and the nitrogen as the carrier gas into the reactor, raising the temperature of the reactor to 400°C or above (such as 550°C), and introducing air for calcination to complete primary silanization modification of the zeolite molecular sieve; and
(7) repeating steps (2) and (3) for n times.

Optionally, "in situ" in the "in situ chemical vapor deposition method" and the "in situ vapor atomic layer deposition method" refers to catalysis of a coupling conversion reaction of naphtha and CO₂ in the same reactor without the step of removing the modified molecular sieve after the silanization modification of the molecular sieve in the reactor. The in situ vapor silanization method reduces the steps of transportation, loading and washing of a catalyst, and greatly reduces the cost.

Optionally, the silanization reagent used for the silanization modification is selected from at least one of compounds with the following chemical formula: 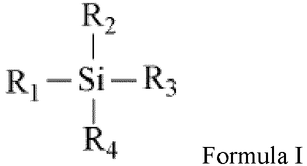 where R₁, R₂, R₃ and R₄ are independently selected from C₁₋₁₀ alkyl and C₁₋₁₀ alkoxyl.

Optionally, according to the silanization reagent used for the silanization modification, at least one of the R₁, the R₂, the R₃ and the R₄ is selected from C₁₋₁₀ alkoxyl.

Optionally, the silanization reagent is selected from at least one of tetraethyl silicate and tetramethyl silicate.

On the other hand, the present application provides a method for producing benzene, toluene and p-xylene by coupling conversion of naphtha and CO₂, where the method includes: (a) preparing a modified molecular sieve catalyst by the above method; and (b) enabling a raw material containing naphtha and CO₂ to contact with the modified molecular sieve catalyst in a reactor for a reaction to produce benzene, toluene and p-xylene.

Optionally, the modified zeolite molecular sieve is a zeolite molecular sieve obtained by metal modification and silanization modification.

Optionally, the modified zeolite molecular sieve is a zeolite molecular sieve obtained by carrying out metal modification first and then carrying out silanization modification.

Optionally, the modified zeolite molecular sieve is a zeolite molecular sieve obtained only by silanization modification.

Optionally, the zeolite molecular sieve catalyst consists of a modified zeolite molecular sieve.

Optionally, the modified zeolite molecular sieve is a zeolite molecular sieve obtained by sequentially carrying out metal modification, silanization modification and in situ water vapor modification.

Specifically, the water vapor modification is carried out in the following steps: introducing water vapor into a reactor where a molecular sieve obtained by in situ silanization modification is placed, and raising the temperature to 700-900°C in a nitrogen atmosphere for treatment for predetermined time.

Optionally, the raw material consists of naphtha and CO₂.

Optionally, the naphtha is selected from at least one of hydrocracked naphtha, catalytic cracked naphtha, raffinate oil, topped oil and direct coal liquefied naphtha.

Optionally, the carbon number distribution of hydrocarbons in the naphtha is in a range of C₄-C₁₂.

Optionally, the reactor is one of a fixed bed reactor, a fluidized bed reactor or a moving bed reactor.

Optionally, conditions for the reaction of the naphtha and the CO₂ are as follows: the reaction temperature is 450-650°C, the reaction pressure is 0.1-3 MPa, the weight hourly space velocity of the naphtha is 0.1-5 h⁻¹, and the weight hourly space velocity of the CO₂ is 0.1-5 h⁻¹.

Optionally, the reaction temperature is selected from any value of 450°C, 500°C, 550°C and 650°C, or a range value determined by any two of the values.

Optionally, the reaction pressure is selected from any value of 0.1 MPa, 1 MPa and 3 MPa, or a range value determined by two of the values.

Optionally, the weight hourly space velocity of the naphtha is selected from any value of 0.1 h⁻¹, 1 h⁻¹ and 5 h⁻¹, or a range value determined by any two of the values.

Optionally, the weight hourly space velocity of the CO₂ is selected from any value of 0.1 h⁻¹, 0.3 h⁻¹ and 5 h⁻¹, or a range value determined by any two of the values.

Optionally, the reaction time is 30-120 minutes.

Optionally, the mass ratio of the CO₂ to the naphtha is 0.8:0.27 to 0.8:1;
and optionally, the mass ratio of the CO₂ to the naphtha is any value of 0.8:0.27, 0.8:1 and 1:1, or a range value determined by any two of the values.

Optionally, the zeolite molecular sieve catalyst further includes a binder.

In the present application, a method for preparing a zeolite molecular sieve catalyst used in a fluidized bed includes: uniformly mixing a metal-modified zeolite molecular sieve with a binder in water, carrying out beating, milling and defoaming to obtain slurry, and subjecting the obtained slurry to conventional spray drying, molding and calcination to obtain the zeolite molecular sieve used in the fluidized bed; where the binder includes an amorphous binder containing aluminum or silicon, preferably pseudo-boehmite or silica sol.

Specifically, the method for producing BTPX by coupling conversion of naphtha and CO₂ includes:
(i) loading a zeolite molecular sieve catalyst into a reactor, carrying out pretreatment with an inert gas such as nitrogen at a predetermined temperature, and adjusting the temperature to a reaction temperature in a nitrogen atmosphere;
(ii) feeding naphtha and CO₂, where the naphtha is fed by a micro-feed pump, the flow and feeding amount of the CO₂ are controlled by a flow meter, and the reaction pressure is controlled within a predetermined range; and
(iii) after a reaction of the naphtha and the CO₂ for predetermined time, analyzing products by gas chromatography.

The "hydrocracked naphtha" of the present application refers to heavy naphtha produced by a hydrocracking reaction of heavy oil.

The "catalytic cracked naphtha" of the present application refers to naphtha produced by catalytic cracking of vacuum gas oil and atmospheric residue.

The "raffinate oil" of the present application refers to distillate remaining after aromatic hydrocarbon extraction of a catalytic reforming product rich in aromatic hydrocarbons.

The "topped oil" of the present application refers to a light fraction with a boiling point of lower than 60°C obtained by distillation of straight-run gasoline.

The "direct coal liquefied naphtha" of the present application refers to naphtha produced by a direct coal liquefaction device.

In the present application, the coupling conversion of naphtha and CO₂ to produce aromatic hydrocarbons refers to a reaction of the CO₂ as a raw material and the naphtha to produce aromatic hydrocarbons.

On another hand, the present application provides a method for silanization modification of a molecular sieve catalyst, where the method includes the following steps:
(1-1) placing a metal-modified or unmodified zeolite molecular sieve solid in a reactor;
(2-1) introducing a material A containing a silanization reagent into the reactor at n times, where the introduced amount of the silanization reagent each time is 0.03-0.06 g/g solid, the silanization reagent is gaseous in the reactor, and the n is in a value range of 3-6; and
(3-1) stopping the introduction of the material A into the reactor, raising the temperature of the reactor to 400°C or above, and introducing air for calcination.

Specifically, the silanization modification is carried out by using an in situ vapor atomic layer deposition method, which includes the following steps:
(1-1') placing a metal-modified or unmodified zeolite molecular sieve solid in a reactor;
(2-1') introducing nitrogen as a carrier gas into a saturated flask filled with a silanization agent, introducing the carrier gas carrying the silanization agent into the reactor continuously for predetermined time, carrying out activation treatment with nitrogen under high temperature conditions (such as 450-550°C), and then carrying out cooling to a silanization modification temperature in a nitrogen atmosphere (the temperature ensures that the silanization reagent is in a gaseous state in the reactor);
(3-1') stopping the introduction of the silanization reagent and the nitrogen as the carrier gas into the reactor, raising the temperature of the reactor to 400°C or above (such as 550°C), and introducing air for calcination to complete primary silanization modification of the zeolite molecular sieve; and
(4-1') repeating steps (2) and (3) for n times.

On another hand, the present application provides a method for modifying a molecular sieve catalyst, where the method includes the following steps:
(1'-1) preparing a soluble metal salt aqueous solution;
(2'-1) placing a zeolite molecular sieve in the soluble metal salt aqueous solution, and impregnating the same at a temperature of 60-100°C; and
(3'-1) draining the modified molecular sieve obtained in step (2), followed by drying and calcination;
(4'-1) placing the metal-modified molecular sieve obtained in step (3) in a reactor;
(5'-1) introducing a material A containing a silanization reagent into the reactor at n times, where the introduced amount of the silanization reagent each time is 0.03-0.06 g/g solid, the silanization reagent is gaseous in the reactor, and the n is in a value range of 3-6; and
(6'-1) stopping the introduction of the material A into the reactor, raising the temperature of the reactor to 400°C or above, and introducing air for calcination to obtain a molecular sieve modified by metal modification first and then vapor silanization modification.

Optionally, the solid-liquid ratio of the modified zeolite molecular sieve to the soluble metal salt aqueous solution is 1/10 to 1/1 (mass ratio), and the mass concentration of a metal salt in the soluble metal salt aqueous solution is 10%-30%; the impregnating time is 2-10 hours; the step of drying is carried out in an air atmosphere at 100-150°C; and the step of calcination is carried out in an air atmosphere at 500-700°C.

Optionally, in step (1), the soluble metal salt aqueous solution is heated to any value or a range value determined by any two values of the following temperatures: 60°C, 70°C, 80°C, 90°C and 100°C.

Optionally, in step (2), the impregnating time is any value or a range value determined by any two values of the following times: 2 hours, 4 hours, 6 hours, 8 hours and 10 hours.

Optionally, the zeolite molecular sieve is an HZSM-5 hydrogen type molecular sieve.

Optionally, a metal in the metal-modified zeolite molecular sieve is selected from at least one of La, Zn, Ga, Fe, Mo and Cr metals.

Optionally, the silanization reagent used for the silanization modification is selected from at least one of compounds with the following chemical formula: 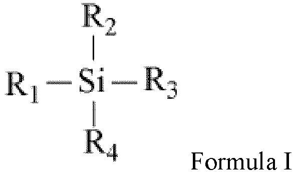 where R₁, R₂, R₃ and R₄ are independently selected from C₁₋₁₀ alkyl and C₁₋₁₀ alkoxyl.

Optionally, at least one of the R₁, the R₂, the R₃ and the R₄ is selected from C₁₋₁₀ alkoxyl.

Optionally, the silanization reagent is selected from at least one of tetraethyl silicate and tetramethyl silicate.

The present application has the following beneficial effects.
(1) The present application provides a new technical route for large-scale production of benzene, toluene and p-xylene (BTPX) by means of CO₂, and the method overcomes the defects of limited hydrogen resources and high costs of existing technologies.
(2) In the present application, with a modified HZSM-5 molecular sieve as an active component of a catalyst, the selectivity of BTPX in hydrocarbon products is as high as 75.08%, and the selectivity of p-xylene in xylene is 92% or above.
(3) In the present application, compared with the use of an HZSM-5 molecular sieve obtained only by metal modification as an active component of a catalyst, the use of an HZSM-5 molecular sieve obtained by metal modification and silanization modification as an active component of a catalyst can achieve higher selectivity of BTPX and higher selectivity of p-xylene in xylene.
(4) In the present application, compared with the use of an in situ chemical vapor deposition method for silanization, the use of an in situ vapor atomic layer deposition method for silanization modification has the advantages that the modified HZSM-5 molecular sieve as an active component can achieve higher selectivity of BTPX and higher selectivity of p-xylene in xylene.

### DESCRIPTION OF THE EMBODIMENTS

The present application is described in detail below in conjunction with examples, but the present application is not limited to the examples.

Endpoints and any values of ranges disclosed in the present application are not limited to the exact ranges or values, and these ranges or values should be understood as including approximate ranges or values. For numeric ranges, endpoint values and individual point values of each range may be combined with each other to obtain one or more new numeric ranges, and these numeric ranges should be considered to be specifically disclosed herein.

The present application is described in detail below in conjunction with examples, but the present application is not limited to the examples.

Unless otherwise specified, raw materials used in the examples of the present application are purchased by commercial ways or prepared by known methods. An HZSM-5 zeolite molecular sieve used in the examples was purchased from Nankai Catalyst Factory.

Unless otherwise specified, analytical methods used in the examples are implemented with conventional setup of instruments or equipment and are conventional analytical methods.

In the examples of the present application, the type of naphtha is direct coal liquefied naphtha, which includes specific components as shown in the following table.

### Composition of direct coal liquefied naphtha

| Carbon number | N-alkanes | Isoalkanes | Cycloalkanes | Aromatic hydrocarbons |
|---|---|---|---|---|
| 6 | 0.03 | 0.00 | 0.00 | 0.00 |
| 7 | 3.76 | 0.71 | 31.85 | 1.60 |
| 8 | 9.36 | 2.62 | 27.53 | 1.94 |
| 9 | 2.03 | 2.44 | 13.88 | 0.40 |
| 10 | 0.15 | 0.75 | 0.74 | 0.07 |
| 11 | 0.01 | 0.03 | 0.10 | 0.00 |
| Total | 15.34 | 6.55 | 74.10 | 4.01 |

In the examples of the present application, the inner diameter of a fixed bed reactor is 1.5 cm; and the inner diameter of a fixed fluidized bed reactor is 3 cm.

In the examples of the present application, only hydrocarbon products are listed, and other products obtained by a reaction of naphtha and CO₂ are not listed.

### Example 1 Preparation of a zinc-modified HZSM-5 molecular sieve molded sample used in a fixed bed

100 g of an HZSM-5 zeolite molecular sieve (Nankai Catalyst Factory, Si/Al=15) was placed in a 10 wt% zinc nitrate aqueous solution, where the mass ratio (namely solid-liquid ratio) of the HZSM-5 zeolite molecular sieve to the zinc nitrate aqueous solution was 1/10. The molecular sieve was impregnated at 80°C for 6 hours, drained, dried in an air atmosphere at 120°C for 4 hours, and then calcined in an air atmosphere at 550°C for 4 hours to obtain a [Zn]HZSM-5 molecular sieve sample. Then, the sample was subjected to pressing molding, crushed and sieved to obtain molded molecular sieve particles with a particle size of 40-60 mesh, recorded as FX-[Zn]HZSM-5.

### Example 2 Preparation of a gallium-modified HZSM-5 molecular sieve molded sample used in a fixed bed

100 g of an HZSM-5 zeolite molecular sieve (Nankai Catalyst Factory, Si/Al=15) was placed in a 10 wt% gallium nitrate aqueous solution, where the mass ratio (namely solid-liquid ratio) of the HZSM-5 zeolite molecular sieve to the gallium nitrate aqueous solution was 1/10. The molecular sieve was impregnated at 80°C for 6 hours, drained, dried in an air atmosphere at 120°C for 4 hours, and then calcined in an air atmosphere at 550°C for 4 hours to obtain a [Ga]HZSM-5 molecular sieve sample. Then, the sample was subjected to pressing molding, crushed and sieved to obtain molded molecular sieve particles with a particle size of 40-60 mesh, recorded as FX-[Ga]HZSM-5.

### Example 3 Preparation of a lanthanum-modified HZSM-5 molecular sieve molded sample used in a fixed bed

100 g of an HZSM-5 zeolite molecular sieve (Nankai Catalyst Factory, Si/Al=15) was placed in a 10 wt% lanthanum nitrate aqueous solution, where the mass ratio (namely solid-liquid ratio) of the HZSM-5 zeolite molecular sieve to the lanthanum nitrate aqueous solution was 1/10. The molecular sieve was impregnated at 90°C for 4 hours, drained, dried in an air atmosphere at 120°C for 4 hours, and then calcined in an air atmosphere at 550°C for 4 hours to obtain a [La]HZSM-5 molecular sieve sample. Then, the sample was subjected to pressing molding, crushed and sieved to obtain molded molecular sieve particles with a particle size of 40-60 mesh, recorded as FX-[La]HZSM-5.

### Example 4 Preparation of an iron-modified HZSM-5 molecular sieve molded sample used in a fixed bed

100 g of an HZSM-5 zeolite molecular sieve (Nankai Catalyst Factory, Si/Al=15) was placed in a 10 wt% ferric nitrate aqueous solution, where the mass ratio (namely solid-liquid ratio) of the HZSM-5 zeolite molecular sieve to the ferric nitrate aqueous solution was 1/10. The molecular sieve was impregnated at 70°C for 8 hours, drained, dried in an air atmosphere at 120°C for 4 hours, and then calcined in an air atmosphere at 550°C for 4 hours to obtain a [Fe]HZSM-5 molecular sieve sample. Then, the sample was subjected to pressing molding, crushed and sieved to obtain molded molecular sieve particles with a particle size of 40-60 mesh, recorded as FX-[Fe]HZSM-5.

### Example 5 Preparation of a chromium-modified HZSM-5 molecular sieve molded sample used in a fixed bed

100 g of an HZSM-5 zeolite molecular sieve (Nankai Catalyst Factory, Si/Al=15) was placed in a 10 wt% chromium nitrate aqueous solution, where the mass ratio (namely solid-liquid ratio) of the HZSM-5 zeolite molecular sieve to the chromium nitrate aqueous solution was 1/10. The molecular sieve was impregnated at 70°C for 8 hours, drained, dried in an air atmosphere at 120°C for 4 hours, and then calcined in an air atmosphere at 550°C for 4 hours to obtain a [Cr]HZSM-5 molecular sieve sample. Then, the sample was subjected to pressing molding, crushed and sieved to obtain molded molecular sieve particles with a particle size of 40-60 mesh, recorded as FX-[Cr]HZSM-5.

### Example 6 Preparation of a zinc-modified HZSM-5 molecular sieve molded sample used in a fluidized bed

100 g of the [Zn]HZSM-5 molecular sieve sample prepared in Example 1 was mixed with an amorphous binder containing aluminum or silicon for spray drying and molding. Specific steps are as follows.

The [Zn]HZSM-5 molecular sieve sample, pseudo-boehmite, silica sol, xanthan gum (biological gum) and water were uniformly mixed, followed by beating, milling and defoaming to obtain slurry. The slurry includes the following parts by weight of components:

| | |
|---|---|
| [Zn]HZSM-5 | 35 parts by weight, |
| Al₂O₃ | 20 parts by weight, |
| SiO₂ | 45 parts by weight, |
| H₂O | 240 parts by weight, and |
| xanthan gum | 1 part by weight. |

The obtained slurry was subjected to spray drying and molding to obtain a microsphere particle sample with the particle size distribution of 20-100 µm. Then, the microsphere particle sample was calcined in a Muffle furnace at 550°C for 3 hours to obtain a [Zn]HZSM-5 molded molecular sieve with an abrasion index of 1.2, recorded as FL-[Zn]HZSM-5.

### Example 7 Preparation of a zinc-gallium composite modified HZSM-5 molecular sieve molded sample used in a fixed bed

100 g of an HZSM-5 zeolite molecular sieve (Nankai Catalyst Factory, Si/Al=15) was placed in a 10 wt% mixed aqueous solution of zinc nitrate and gallium nitrate, where the mass ratio of the zinc nitrate to the gallium nitrate was 1/1, and the mass ratio (namely solid-liquid ratio) of the HZSM-5 zeolite molecular sieve to the mixed aqueous solution of the zinc nitrate and the gallium nitrate was 1/10. The molecular sieve was impregnated at 80°C for 6 hours, drained, dried in an air atmosphere at 120°C for 4 hours, and then calcined in an air atmosphere at 550°C for 4 hours to obtain a [Zn,Ga]HZSM-5 molecular sieve sample. Then, the sample was subjected to pressing molding, crushed and sieved to obtain molded molecular sieve particles with a particle size of 40-60 mesh, recorded as FX-[Zn,Ga]HZSM-5.

### Example 8 Preparation of a zinc-modified HZSM-5 molecular sieve molded sample used in a fixed bed

100 g of an HZSM-5 zeolite molecular sieve (Nankai Catalyst Factory, Si/Al=15) was placed in a 10 wt% zinc nitrate aqueous solution, where the mass ratio (namely solid-liquid ratio) of the HZSM-5 zeolite molecular sieve to the zinc nitrate aqueous solution was the same as that in Example 1. The molecular sieve was impregnated at room temperature (20°C) for 6 hours, drained, dried in an air atmosphere at 120°C for 4 hours, and then calcined in an air atmosphere at 550°C for 4 hours to obtain a [Zn]HZSM-5 molecular sieve sample. Then, the sample was subjected to pressing molding, crushed and sieved to obtain molded molecular sieve particles with a particle size of 40-60 mesh, recorded as FX-[Zn]HZSM-5-R.

### Example 9 Preparation of a zinc-modified HZSM-5 molecular sieve molded sample used in a fixed bed

100 g of an HZSM-5 zeolite molecular sieve (Nankai Catalyst Factory, Si/Al=15) was placed in a 10 wt% zinc nitrate aqueous solution, where the mass ratio (namely solid-liquid ratio) of the HZSM-5 zeolite molecular sieve to the zinc nitrate aqueous solution was 1/1. The molecular sieve was impregnated at 80°C for 6 hours, drained, dried in an air atmosphere at 120°C for 4 hours, and then calcined in an air atmosphere at 550°C for 4 hours to obtain a [Zn]HZSM-5 molecular sieve sample. Then, the sample was subjected to pressing molding, crushed and sieved to obtain molded molecular sieve particles with a particle size of 40-60 mesh, recorded as FX-[Zn]HZSM-5-A.

### Example 10 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5 catalyst prepared in Example 1 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was 0.2 g/g the catalyst. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-1.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 1.

**Table 1 Evaluation of reaction performance of a catalyst in Example 10**

| | | |
|---|---|---|
| Conversion rate of naphtha (wt%) | | 80.12 |
| Conversion rate of CO₂ (wt%) | | 31.13 |
| Selectivity of ethylene and propylene in hydrocarbon products (wt%) | | 12.61 |
| Selectivity of benzene, toluene and PX in hydrocarbon products (wt%) | | 69.06 |
| Selectivity of aromatic hydrocarbons in hydrocarbon products (wt%) | | 73.73 |
| Selectivity of PX in xylene products (wt%) | | 95.50 |

| Composition of hydrocarbon products (wt%) | | |
|---|---|---|
| | Methane | 2.29 |
| | Ethylene | 5.4 |
| | Ethane | 2.93 |
| | Propylene | 7.21 |
| | Propane | 3.26 |
| | C₄ | 5.18 |
| | Benzene | 19.3 |
| | Toluene | 37.45 |
| | Ethylbenzene | 1.34 |
| | P-xylene | 12.31 |
| | M-xylene | 0.39 |
| | O-xylene | 0.19 |
| | C₈₊ aromatic hydrocarbons | 2.75 |

### Example 10-1 Preparation of a catalyst used for producing benzene, toluene and p-xylene and reaction evaluation

The reaction performance of a catalyst used for coupling conversion of naphtha and CO₂ was evaluated in a micro-fixed bed reactor. Evaluation conditions are as follows. 5 g of the FX-[Zn]HZSM-5 prepared in Example 1 was loaded into a fixed bed reactor, and treated with nitrogen at 50 ml/min at 550°C for 1 hour.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 1-1.

**Table 1-1 Evaluation of reaction performance of a catalyst in Example 10-1**

| | | |
|---|---|---|
| Conversion rate of naphtha (wt%) | | 88.48 |
| Conversion rate of CO₂ (wt%) | | 35.53 |
| Selectivity of ethylene and propylene in hydrocarbon products (wt%) | | 7.32 |
| Selectivity of benzene, toluene and PX in hydrocarbon products (wt%) | | 63.00 |
| Selectivity of aromatic hydrocarbons in hydrocarbon products (wt%) | | 77.60 |
| Selectivity of PX in xylene products (wt%) | | 24.30 |

| Composition of hydrocarbon products (wt%) | | |
|---|---|---|
| | Methane | 3.81 |
| | Ethylene | 3.97 |
| | Ethane | 5.03 |
| | Propylene | 3.35 |
| | Propane | 5.51 |
| | C₄ | 0.73 |
| | Benzene | 25.93 |
| | Toluene | 34.53 |
| | Ethylbenzene | 0.43 |
| | P-xylene | 2.54 |
| | M-xylene | 5.47 |
| | 0-xylene | 2.44 |
| | C₈₊ aromatic hydrocarbons | 6.26 |

As can be seen by comparing Example 10 and Example 10-1, compared with the use of the HZSM-5 molecular sieve obtained only by metal modification as an active component of the catalyst, the use of the HZSM-5 molecular sieve obtained by metal modification and silanization modification as an active component of the catalyst can achieve higher selectivity of BTPX and higher selectivity of p-xylene in xylene.

### Example 11 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Ga]HZSM-5 catalyst prepared in Example 2 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-2.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 2.

**Table 2 Evaluation of reaction performance of a catalyst in Example 11**

| | | |
|---|---|---|
| Conversion rate of naphtha (wt%) | | 79.78 |
| Conversion rate of CO₂ (wt%) | | 29.97 |
| Selectivity of ethylene and propylene in hydrocarbon products (wt%) | | 9.22 |
| Selectivity of benzene, toluene and PX in hydrocarbon products (wt%) | | 69.95 |
| Selectivity of aromatic hydrocarbons in hydrocarbon products (wt%) | | 74.55 |
| Selectivity of PX in xylene products (wt%) | | 96.13 |

| Composition of hydrocarbon products (wt%) | | |
|---|---|---|
| | Methane | 1.67 |
| | Ethylene | 5.01 |
| | Ethane | 2.68 |
| | Propylene | 6.21 |
| | Propane | 4.3 |
| | C₄ | 5.58 |
| | Benzene | 18.19 |
| | Toluene | 38.35 |
| | Ethylbenzene | 1.45 |
| | P-xylene | 13.41 |
| | M-xylene | 0.37 |
| | O-xylene | 0.17 |
| | C₈₊ aromatic hydrocarbons | 2.61 |

### Example 12 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[La]HZSM-5 catalyst prepared in Example 3 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-3.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 3.

**Table 3 Evaluation of reaction performance of a catalyst in Example 12**

| | | |
|---|---|---|
| Conversion rate of naphtha (wt%) | | 78.41 |
| Conversion rate of CO₂ (wt%) | | 25.83 |
| Selectivity of ethylene and propylene in hydrocarbon products (wt%) | | 12.46 |
| Selectivity of benzene, toluene and PX in hydrocarbon products (wt%) | | 67.89 |
| Selectivity of aromatic hydrocarbons in hydrocarbon products (wt%) | | 72.87 |
| Selectivity of PX in xylene products (wt%) | | 95.58 |

| Composition of hydrocarbon products (wt%) | | |
|---|---|---|
| | Methane | 3.37 |
| | Ethylene | 5.53 |
| | Ethane | 4.12 |
| | Propylene | 6.93 |
| | Propane | 3.22 |
| | C₄ | 3.96 |
| | Benzene | 20.58 |
| | Toluene | 36.5 |
| | Ethylbenzene | 1.15 |
| | P-xylene | 10.81 |
| | M-xylene | 0.38 |
| | O-xylene | 0.12 |
| | C₈₊ aromatic hydrocarbons | 3.33 |

### Example 13 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Fe]HZSM-5 catalyst prepared in Example 4 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-4.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 4.

**Table 4 Evaluation of reaction performance of a catalyst in Example 13**

| | | |
|---|---|---|
| Conversion rate of naphtha (wt%) | | 77.80 |
| Conversion rate of CO₂ (wt%) | | 24.09 |
| Selectivity of ethylene and propylene in hydrocarbon products (wt%) | | 12.06 |
| Selectivity of benzene, toluene and PX in hydrocarbon products (wt%) | | 66.66 |
| Selectivity of aromatic hydrocarbons in hydrocarbon products (wt%) | | 71.58 |
| Selectivity of PX in xylene products (wt%) | | 95.93 |

| Composition of hydrocarbon products (wt%) | | |
|---|---|---|
| | Methane | 2.82 |
| | Ethylene | 4.95 |
| | Ethane | 2.82 |
| | Propylene | 7.11 |
| | Propane | 4.16 |
| | C₄ | 6.56 |
| | Benzene | 17.12 |
| | Toluene | 36.58 |
| | Ethylbenzene | 1.46 |
| | P-xylene | 12.96 |
| | M-xylene | 0.41 |
| | O-xylene | 0.14 |
| | C₈₊ aromatic hydrocarbons | 2.91 |

### Example 14 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Cr]HZSM-5 catalyst prepared in Example 5 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-5.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 5.

**Table 5 Evaluation of reaction performance of a catalyst in Example 14**

| | | |
|---|---|---|
| Conversion rate of naphtha (wt%) | | 79.90 |
| Conversion rate of CO₂ (wt%) | | 29.57 |
| Selectivity of ethylene and propylene in hydrocarbon products (wt%) | | 13.67 |
| Selectivity of benzene, toluene and PX in hydrocarbon products (wt%) | | 67.74 |
| Selectivity of aromatic hydrocarbons in hydrocarbon products (wt%) | | 72.70 |
| Selectivity of PX in xylene products (wt%) | | 95.68 |

| Composition of hydrocarbon products (wt%) | | |
|---|---|---|
| | Methane | 2.03 |
| | Ethylene | 5.28 |
| | Ethane | 2.72 |
| | Propylene | 8.39 |
| | Propane | 3.09 |
| | C₄ | 5.79 |
| | Benzene | 18.32 |
| | Toluene | 36.57 |
| | Ethylbenzene | 1.59 |
| | P-xylene | 12.85 |
| | M-xylene | 0.42 |
| | O-xylene | 0.16 |
| | C₈₊ aromatic hydrocarbons | 2.79 |

### Example 15 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed fluidized bed reactor. Conditions for on-line preparation of the catalyst are as follows. 10 g of the FX-[Zn]HZSM-5 catalyst prepared in Example 6 was loaded into a fixed fluidized bed reactor, treated with nitrogen at 50 ml/min at 550°C for 1 hour, and then cooled to 300°C in a nitrogen atmosphere. In the nitrogen atmosphere (controlled by a mass flow meter, 200 mL/min), tetraethyl silicate was pumped into the reactor at a weight hourly space velocity of 0.2 h⁻¹ at normal pressure. After feeding for 75 minutes, the feeding was stopped, where the introduced amount of the tetraethyl silicate was 1.25 times of that in Example 9. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FLNCC-1. The purpose of using 10 g of the FL-[Zn]HZSM-5 molecular sieve molded sample in this example was only to meet requirements of the micro-fixed fluidized bed reactor, the amount (10 g) enables the catalyst to stay in a fluidized state, and the amount of 5 g cannot enable the catalyst to stay in a fluidized state. In a case of using the micro-fixed fluidized bed reactor, the feeding time was 75 min for silanization modification, which was only used to carry out silanization modification to the same degree as that in Example 9.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 6.

**Table 6 Evaluation of reaction performance of a catalyst in Example 15**

| | | |
|---|---|---|
| Conversion rate of naphtha (wt%) | | 79.27 |
| Conversion rate of CO₂ (wt%) | | 29.29 |
| Selectivity of ethylene and propylene in hydrocarbon products (wt%) | | 11.63 |
| Selectivity of BTX in hydrocarbon products (wt%) | | 67.86 |
| Selectivity of aromatic hydrocarbons in hydrocarbon products (wt%) | | 72.99 |
| Selectivity of PX in xylene products | | 96.13 |

| Composition of hydrocarbon products (wt%) | | |
|---|---|---|
| | Methane | 2.74 |
| | Ethylene | 4.57 |
| | Ethane | 2.52 |
| | Propylene | 7.06 |
| | Propane | 3.66 |
| | C₄ | 6.46 |
| | Benzene | 17.08 |
| | Toluene | 37.13 |
| | Ethylbenzene | 1.58 |
| | P-xylene | 13.65 |
| | M-xylene | 0.37 |
| | O-xylene | 0.18 |
| | C₈₊ aromatic hydrocarbons | 3.00 |

### Example 16 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5 catalyst prepared in Example 1 was loaded into a fixed bed reactor, treated with nitrogen at 50 ml/min at 550°C for 1 hour, and then cooled to 300°C in a nitrogen atmosphere. Then, a vapor atomic layer deposition method was used to carry out modification with a silanization reagent, specifically including the following steps: (1) introducing nitrogen (controlled by a mass flow meter, 200 mL/min) into a saturated flask filled with tetraethyl silicate (temperature 15°C), then introducing the same into a reactor, namely introducing the nitrogen carrying the tetraethyl silicate into the reactor, and after feeding for 5 minutes, stopping the feeding; (2) carrying out purging with nitrogen, and raising the temperature to 550°C, followed by calcination in an air atmosphere for 1 hour; and (3) repeating steps (1) and (2) for 3 times to obtain a fixed bed catalyst used for coupling conversion of naphtha and CO₂ to produce benzene, toluene and p-xylene, named as FXNCC-6, where the amount of the tetraethyl silicate introduced for 4 times was equivalent to the amount introduced for one time in Example 10.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 7.

**Table 7 Evaluation of reaction performance of a catalyst in Example 16**

| | | |
|---|---|---|
| Conversion rate of naphtha (wt%) | | 86.57 |
| Conversion rate of CO₂ (wt%) | | 34.16 |
| Selectivity of ethylene and propylene in hydrocarbon products (wt%) | | 8.78 |
| Selectivity of BTX in hydrocarbon products (wt%) | | 74.50 |
| Selectivity of aromatic hydrocarbons in hydrocarbon products (wt%) | | 78.48 |
| Selectivity of PX in xylene products | | 97.08 |

| Composition of hydrocarbon products (wt%) | | |
|---|---|---|
| | Methane | 2.45 |
| | Ethylene | 4.05 |
| | Ethane | 3.23 |
| | Propylene | 4.73 |
| | Propane | 3.91 |
| | C₄ | 3.15 |
| | Benzene | 20.31 |
| | Toluene | 39.17 |
| | Ethylbenzene | 0.71 |
| | P-xylene | 15.02 |
| | M-xylene | 0.32 |
| | O-xylene | 0.13 |
| | C₈₊ aromatic hydrocarbons | 2.82 |

### Example 17 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn,Ga]HZSM-5 catalyst prepared in Example 7 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-7.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 8.

**Table 8 Evaluation of reaction performance of a catalyst in Example 17**

| | | |
|---|---|---|
| Conversion rate of naphtha (wt%) | | 81.31 |
| Conversion rate of CO₂ (wt%) | | 30.95 |
| Selectivity of ethylene and propylene in hydrocarbon products (wt%) | | 10.24 |
| Selectivity of benzene, toluene and PX in hydrocarbon products (wt%) | | 71.37 |
| Selectivity of aromatic hydrocarbons in hydrocarbon products (wt%) | | 75.91 |
| Selectivity of PX in xylene products (wt%) | | 95.72 |

| Composition of hydrocarbon products (wt%) | | |
|---|---|---|
| | Methane | 2.57 |
| | Ethylene | 4.82 |
| | Ethane | 3.11 |
| | Propylene | 5.42 |
| | Propane | 4.29 |
| | C₄ | 3.89 |
| | Benzene | 20.47 |
| | Toluene | 36.83 |
| | Ethylbenzene | 0.94 |
| | P-xylene | 14.07 |
| | M-xylene | 0.40 |
| | O-xylene | 0.23 |
| | C₈₊ aromatic hydrocarbons | 2.96 |

### Example 18 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5-R catalyst prepared in Example 8 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-8.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 9.

**Table 9 Evaluation of reaction performance of a catalyst in Example 18**

| | | |
|---|---|---|
| Conversion rate of naphtha (wt%) | | 78.37 |
| Conversion rate of CO₂ (wt%) | | 30.81 |
| Selectivity of ethylene and propylene in hydrocarbon products (wt%) | | 15.99 |
| Selectivity of benzene, toluene and PX in hydrocarbon products (wt%) | | 62.62 |
| Selectivity of aromatic hydrocarbons in hydrocarbon products (wt%) | | 67.38 |
| Selectivity of PX in xylene products (wt%) | | 95.39 |

| Composition of hydrocarbon products (wt%) | | |
|---|---|---|
| | Methane | 2.34 |
| | Ethylene | 6.46 |
| | Ethane | 2.96 |
| | Propylene | 9.53 |
| | Propane | 3.29 |
| | C₄ | 8.06 |
| | Benzene | 15.29 |
| | Toluene | 34.58 |
| | Ethylbenzene | 1.35 |
| | P-xylene | 12.75 |
| | M-xylene | 0.40 |
| | O-xylene | 0.21 |
| | C₈₊ aromatic hydrocarbons | 2.78 |

Compared with the selectivity of BTPX in Example 10, the HZSM-5 molecular sieve obtained by metal modification using a high temperature hydrothermal method can achieve higher selectivity of BTPX than that obtained by metal modification using a room temperature impregnation method.

### Example 19 Preparation of a ZSM-5 catalyst modified with a silanization reagent used in a fixed bed

A catalyst used for coupling conversion of naphtha and CO₂ to produce benzene, toluene and p-xylene was prepared on-line in a micro-fixed bed reactor. Conditions for on-line preparation of the catalyst are as follows. 5 g of a 40- to 60-mesh HZSM-5 molecular sieve catalyst was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-9.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 9-1.

**Table 9-1 Evaluation of reaction performance of a catalyst in Example 19**

| | | |
|---|---|---|
| Conversion rate of naphtha (wt%) | | 80.76 |
| Conversion rate of CO₂ (wt%) | | 5.02 |
| Selectivity of ethylene and propylene in hydrocarbon products (wt%) | | 6.33 |
| Selectivity of benzene, toluene and PX in hydrocarbon products (wt%) | | 56.96 |
| Selectivity of aromatic hydrocarbons in hydrocarbon products (wt%) | | 60.83 |
| Selectivity of PX in xylene products (wt%) | | 96.24 |

| Composition of hydrocarbon products (wt%) | | |
|---|---|---|
| | Methane | 15.04 |
| | Ethylene | 3.56 |
| | Ethane | 9.44 |
| | Propylene | 2.77 |
| | Propane | 5.31 |
| | C₄ | 3.05 |
| | Benzene | 20.36 |
| | Toluene | 24.33 |
| | Ethylbenzene | 0.64 |
| | P-xylene | 12.27 |
| | M-xylene | 0.32 |
| | O-xylene | 0.16 |
| | C₈₊ aromatic hydrocarbons | 2.75 |

### Example 20 Preparation of a catalyst used for producing benzene, toluene and p-xylene and reaction evaluation

5 g of the FXNCC-9 catalyst prepared in Example 19 was placed in a 10 wt% lanthanum nitrate aqueous solution, where the solid-liquid ratio of the HZSM-5 zeolite molecular sieve used in the FXNCC-9 catalyst to the lanthanum nitrate aqueous solution was the same as that in Example 3. The catalyst was impregnated at 90°C for 4 hours, drained, dried in an air atmosphere at 120°C for 4 hours, and then calcined in an air atmosphere at 550°C for 4 hours to obtain a catalyst sample, recorded as FXNCC-10.

The reaction performance of the catalyst used for coupling conversion of naphtha and CO₂ was evaluated in a micro-fixed bed reactor. Evaluation conditions are as follows. 5 g of the FXNCC-10 catalyst molded sample (40-60 mesh) was loaded into a fixed bed reactor, and the temperature was raised to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 10.

**Table 10 Evaluation of reaction performance of a catalyst in Example 20**

| | | |
|---|---|---|
| Conversion rate of naphtha (wt%) | | 82.54 |
| Conversion rate of CO₂ (wt%) | | 28.95 |
| Selectivity of ethylene and propylene in hydrocarbon products (wt%) | | 11.80 |
| Selectivity of benzene, toluene and PX in hydrocarbon products (wt%) | | 60.65 |
| Selectivity of aromatic hydrocarbons in hydrocarbon products (wt%) | | 73.16 |
| Selectivity of PX in xylene products (wt%) | | 40.37 |

| Composition of hydrocarbon products (wt%) | | |
|---|---|---|
| | Methane | 3.56 |
| | Ethylene | 5.11 |
| | Ethane | 3.96 |
| | Propylene | 6.69 |
| | Propane | 4.31 |
| | C₄ | 3.21 |
| | Benzene | 21.39 |
| | Toluene | 35.11 |
| | Ethylbenzene | 1.06 |
| | P-xylene | 4.15 |
| | M-xylene | 3.94 |
| | O-xylene | 2.19 |
| | C₈₊ aromatic hydrocarbons | 5.32 |

By comparing with Example 12, the catalyst in this example achieves lower selectivity of BTPX and lower selectivity of p-xylene in xylene. The results indicate that when La is used for metal modification, a molecular sieve obtained by carrying out metal modification first and then carrying out silanization modification can achieve higher selectivity of BTPX and higher selectivity of p-xylene in xylene compared with a molecular sieve obtained by carrying out silanization modification first and then carrying out metal modification.

### Example 21 Preparation of a catalyst used for producing benzene, toluene and p-xylene and reaction evaluation

A catalyst used for conversion of naphtha to produce benzene, toluene and p-xylene was prepared on-line in a micro-fixed bed reactor. Conditions for on-line preparation of the catalyst are as follows. 5 g of the FX-[Zn]HZSM-5 catalyst prepared in Example 1 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours to obtain a fixed bed catalyst used for conversion of naphtha to produce benzene, toluene and p-xylene, named as FXNCC-1.

Then, the temperature was adjusted to a reaction temperature of 550°C in a nitrogen atmosphere. A raw material naphtha was fed by a micro-feed pump, and the flow of N₂ was controlled by a mass flow meter. The mass ratio of the raw material N₂ to the naphtha was 0.51: 1 (namely, an equivalent molar amount of N₂ was used in this example to replace the CO₂ in Example 10), the weight hourly space velocity of the naphtha was 1.0 h⁻¹, the weight hourly space velocity of the N₂ was 0.51 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 11.

**Table 11 Evaluation of reaction performance of a catalyst in Example 21**

| | | |
|---|---|---|
| Conversion rate of naphtha (wt%) | 75.98 | |
| Selectivity of ethylene and propylene in hydrocarbon products (wt%) | 16.13 | |
| Selectivity of benzene, toluene and PX in hydrocarbon products (wt%) | 55.94 | |
| Selectivity of aromatic hydrocarbons in hydrocarbon products (wt%) | | 60.32 |
| Selectivity of PX in xylene products (wt%) | | 95.65 |

| Composition of hydrocarbon products (wt%) | | |
|---|---|---|
| | Methane | 1.68 |
| | Ethylene | 7.6 |
| | Ethane | 5.98 |
| | Propylene | 8.53 |
| | Propane | 9.14 |
| | C₄ | 6.75 |
| | Benzene | 16.91 |
| | Toluene | 29.35 |
| | Ethylbenzene | 1.35 |
| | P-xylene | 9.68 |
| | M-xylene | 0.29 |
| | O-xylene | 0.15 |
| | C₈₊ aromatic hydrocarbons | 2.59 |

The N₂ is used as a diluent in Example 21, and the CO₂ is used as a raw material for reacting with the naphtha in Example 10, which can be seen by comparing the selectivity of aromatic hydrocarbons and the selectivity of BTPX in Examples 10 and 21. Specifically, the selectivity of aromatic hydrocarbons and the selectivity of BTPX in Example 10 are obviously higher than the selectivity of aromatic hydrocarbons in Example 21.

### Example 22 Evaluation of hydrothermal stability of a catalyst

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5 catalyst prepared in Example 1 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours. Then, the mixture was heated to 900°C in the nitrogen atmosphere, and treated for 1 hour in a 100% water vapor atmosphere (WHSV of water was 2 h⁻¹) to obtain a fixed bed catalyst used for coupling conversion of naphtha and CO₂ to produce benzene, toluene and p-xylene, named as FXNCC-11.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 12.

**Table 12 Evaluation of reaction performance of a catalyst in Example 22**

| | | |
|---|---|---|
| Conversion rate of naphtha (wt%) | | 70.87 |
| Conversion rate of CO₂ (wt%) | | 20.96 |
| Selectivity of ethylene and propylene in hydrocarbon products (wt%) | | 17.24 |
| Selectivity of benzene, toluene and PX in hydrocarbon products (wt%) | | 59.28 |
| Selectivity of aromatic hydrocarbons in hydrocarbon products (wt%) | | 62.87 |
| Selectivity of PX in xylene products (wt%) | | 96.43 |

| Composition of hydrocarbon products (wt%) | | |
|---|---|---|
| | Methane | 1.81 |
| | Ethylene | 7.65 |
| | Ethane | 3.77 |
| | Propylene | 9.59 |
| | Propane | 3.94 |
| | C₄ | 10.37 |
| | Benzene | 16.2 |
| | Toluene | 32.56 |
| | Ethylbenzene | 0.69 |
| | P-xylene | 10.52 |
| | M-xylene | 0.26 |
| | O-xylene | 0.13 |
| | C₈₊ aromatic hydrocarbons | 2.51 |

### Example 23 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5-R catalyst prepared in Example 8 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was the same as that in Example 10. A resulting mixture was purged with nitrogen, heated to 550°C, and then calcined in an air atmosphere for 4 hours. Then, the mixture was heated to 900°C in a nitrogen atmosphere, and treated for 1 hour in a 100% water vapor atmosphere (WHSV of water was 2 h⁻¹) to obtain a fixed bed catalyst used for coupling conversion of naphtha and CO₂ to produce benzene, toluene and p-xylene, named as FXNCC-12.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 13.

**Table 13 Evaluation of reaction performance of a catalyst in Example 23**

| | | |
|---|---|---|
| Conversion rate of naphtha (wt%) | | 51.69 |
| Conversion rate of CO₂ (wt%) | | 15.62 |
| Selectivity of ethylene and propylene in hydrocarbon products (wt%) | | 31.54 |
| Selectivity of benzene, toluene and PX in hydrocarbon products (wt%) | | 41.59 |
| Selectivity of aromatic hydrocarbons in hydrocarbon products (wt%) | | 44.90 |
| Selectivity of PX in xylene products (wt%) | | 96.16 |

| Composition of hydrocarbon products (wt%) | | |
|---|---|---|
| | Methane | 1.68 |
| | Ethylene | 13.97 |
| | Ethane | 2.68 |
| | Propylene | 17.57 |
| | Propane | 4.61 |
| | C₄ | 14.59 |
| | Benzene | 12.04 |
| | Toluene | 21.03 |
| | Ethylbenzene | 0.96 |
| | P-xylene | 8.52 |
| | M-xylene | 0.21 |
| | O-xylene | 0.13 |
| | C₈₊ aromatic hydrocarbons | 2.01 |

By comparing Examples 22 and 23, it can be seen that the catalyst obtained after hydrothermal treatment in Example 22 has obviously higher selectivity of aromatic hydrocarbons and higher selectivity of BTPX than the catalyst obtained after hydrothermal treatment in Example 23. Therefore, the hydrothermal stability of the metal-modified HZSM-5 prepared by a high temperature hydrothermal method is obviously better than that of the metal-modified HZSM-5 prepared by a room temperature impregnation method.

### Example 24 Preparation of a catalyst used for producing benzene, toluene and p-xylene and reaction evaluation

A catalyst FXNCC-1 was prepared by the method in Example 10.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 1:1, the weight hourly space velocity of the CO₂ was 5 h⁻¹, the weight hourly space velocity of the naphtha was 5 h⁻¹, and the reaction pressure was 1 MPa. Reaction products were analyzed by on-line Agilent7890 gas chromatography, and sampling was carried out for analysis when a reaction was carried out for 30 minutes. Reaction results are shown in Table 14.

**Table 14 Evaluation of reaction performance of a catalyst in Example 24**

| | | |
|---|---|---|
| Conversion rate of naphtha (wt%) | | 83.19 |
| Conversion rate of CO₂ (wt%) | | 33.61 |
| Selectivity of ethylene and propylene in hydrocarbon products (wt%) | | 7.64 |
| Selectivity of benzene, toluene and PX in hydrocarbon products (wt%) | | 72.07 |
| Selectivity of aromatic hydrocarbons in hydrocarbon products (wt%) | | 77.48 |
| Selectivity of PX in xylene products (wt%) | | 94.76 |

| Composition of hydrocarbon products (wt%) | | |
|---|---|---|
| | Methane | 3.13 |
| | Ethylene | 3.47 |
| | Ethane | 3.09 |
| | Propylene | 4.17 |
| | Propane | 5.01 |
| | C₄ | 3.65 |
| | Benzene | 20.88 |
| | Toluene | 37.81 |
| | Ethylbenzene | 1.54 |
| | P-xylene | 13.38 |
| | M-xylene | 0.48 |
| | O-xylene | 0.26 |
| | C₈₊ aromatic hydrocarbons | 3.13 |

### Example 25 Preparation of a catalyst used for producing benzene, toluene and p-xylene and reaction evaluation

A catalyst FXNCC-1 was prepared by the method in Example 10.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 1:1, the weight hourly space velocity of the CO₂ was 0.1 h⁻¹, the weight hourly space velocity of the naphtha was 0.1 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 15.

**Table 15 Evaluation of reaction performance of a catalyst in Example 25**

| | | |
|---|---|---|
| Conversion rate of naphtha (wt%) | 83.63 | |
| Conversion rate of CO₂ (wt%) | 34.25 | |
| Selectivity of ethylene and propylene in hydrocarbon products (wt%) | 5.88 | |
| Selectivity of benzene, toluene and PX in hydrocarbon products (wt%) | 73.32 | |
| Selectivity of aromatic hydrocarbons in hydrocarbon products (wt%) | 79.69 | |
| Selectivity of PX in xylene products (wt%) | | 92.53 |

| Composition of hydrocarbon products (wt%) | | |
|---|---|---|
| | Methane | 3.61 |
| | Ethylene | 2.63 |
| | Ethane | 3.56 |
| | Propylene | 3.25 |
| | Propane | 4.65 |
| | C₄ | 2.61 |
| | Benzene | 21.61 |
| | Toluene | 38.09 |
| | Ethylbenzene | 1.74 |
| | P-xylene | 13.62 |
| | M-xylene | 0.69 |
| | O-xylene | 0.41 |
| | C₈₊ aromatic hydrocarbons | 3.53 |

### Example 26 Preparation of a zinc-modified HZSM-5 molecular sieve molded sample used in a fixed bed

100 g of an HZSM-5 zeolite molecular sieve (Nankai Catalyst Factory, Si/Al=15) was placed in a 30 wt% zinc nitrate aqueous solution, where the mass ratio (namely solid-liquid ratio) of the HZSM-5 zeolite molecular sieve to the zinc nitrate aqueous solution was 1/10. The molecular sieve was impregnated at 80°C for 4 hours, drained, dried in an air atmosphere at 120°C for 4 hours, and then calcined in an air atmosphere at 550°C for 4 hours to obtain a [Zn]HZSM-5 molecular sieve sample. Then, the sample was subjected to pressing molding, crushed and sieved to obtain molded molecular sieve particles with a particle size of 40-60 mesh, recorded as FX-[Zn]HZSM-5-B.

### Example 27 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5-B catalyst prepared in Example 26 was loaded into a fixed bed reactor, treated with nitrogen at 50 ml/min at 550°C for 1 hour, and then cooled to 300°C in a nitrogen atmosphere. In the nitrogen atmosphere (controlled by a mass flow meter, 100 mL/min), tetraethyl silicate was pumped into the reactor at a weight hourly space velocity of 0.2 h⁻¹ at normal pressure. After feeding for 60 minutes, the feeding was stopped, where the introduced amount of the tetraethyl silicate was 0.2 g/g the catalyst. A resulting mixture was purged with nitrogen, heated to 550°C, and then calcined in an air atmosphere for 4 hours to obtain a fixed bed catalyst used for coupling conversion of naphtha and CO₂ to produce benzene, toluene and p-xylene, named as FXNCC-13.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the CO₂ was 0.8 h⁻¹, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 16.

**Table 16 Evaluation of reaction performance of a catalyst in Example 27**

| | | |
|---|---|---|
| Conversion rate of naphtha (wt%) | | 81.66 |
| Conversion rate of CO₂ (wt%) | | 30.09 |
| Selectivity of ethylene and propylene in hydrocarbon products (wt%) | | 10.61 |
| Selectivity of benzene, toluene and PX in hydrocarbon products (wt%) | | 70.28 |
| Selectivity of aromatic hydrocarbons in hydrocarbon products (wt%) | | 74.71 |
| Selectivity of PX in xylene products (wt%) | | 95.04 |

| Composition of hydrocarbon products (wt%) | | |
|---|---|---|
| | Methane | 2.94 |
| | Ethylene | 4.96 |
| | Ethane | 3.21 |
| | Propylene | 5.65 |
| | Propane | 4.29 |
| | C₄ | 4.24 |
| | Benzene | 19.93 |
| | Toluene | 37.90 |
| | Ethylbenzene | 1.08 |
| | P-xylene | 12.45 |
| | M-xylene | 0.43 |
| | O-xylene | 0.22 |
| | C₈₊ aromatic hydrocarbons | 2.70 |

### Example 28 Preparation of a catalyst used for producing benzene, toluene and p-xylene and reaction evaluation

A catalyst FXNCC-1 was prepared by the method in Example 10.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:0.27, the weight hourly space velocity of the CO₂ was 0.8 h⁻¹, the weight hourly space velocity of the naphtha was 0.27 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 17.

**Table 17 Evaluation of reaction performance of a catalyst in Example 28**

| | | |
|---|---|---|
| Conversion rate of naphtha (wt%) | | 82.73 |
| Conversion rate of CO₂ (wt%) | | 15.31 |
| Selectivity of ethylene and propylene in hydrocarbon products (wt%) | | 10.63 |
| Selectivity of benzene, toluene and PX in hydrocarbon products (wt%) | | 73.07 |
| Selectivity of aromatic hydrocarbons in hydrocarbon products (wt%) | | 77.59 |
| Selectivity of PX in xylene products (wt%) | | 95.45 |

| Composition of hydrocarbon products (wt%) | | |
|---|---|---|
| | Methane | 2.07 |
| | Ethylene | 4.72 |
| | Ethane | 2.55 |
| | Propylene | 5.91 |
| | Propane | 3.15 |
| | C₄ | 4.01 |
| | Benzene | 21.06 |
| | Toluene | 38.99 |
| | Ethylbenzene | 1.21 |
| | P-xylene | 13.02 |
| | M-xylene | 0.41 |
| | O-xylene | 0.21 |
| | C₈₊ aromatic hydrocarbons | 2.69 |

### Example 29 Preparation of a catalyst used for producing benzene, toluene and p-xylene and reaction evaluation

A catalyst used for coupling conversion of naphtha and CO₂ to produce benzene, toluene and p-xylene was prepared on-line in a micro-fixed bed reactor. Conditions for on-line preparation of the catalyst are as follows. 5 g of the FX-[Zn]HZSM-5 catalyst prepared in Example 1 was loaded into a fixed bed reactor, treated with nitrogen at 50 ml/min at 550°C for 1 hour, and then cooled to 300°C in a nitrogen atmosphere. Then, a vapor atomic layer deposition method was used to carry out modification with a silanization reagent, specifically including the following steps: (1) introducing nitrogen (controlled by a mass flow meter, 200 mL/min) into a saturated flask filled with tetraethyl silicate (temperature 10°C), then introducing the same into a reactor, namely introducing the nitrogen carrying the tetraethyl silicate into the reactor, and after feeding for 5 minutes, stopping the feeding; (2) carrying out purging with nitrogen, and raising the temperature to 550°C, followed by calcination in an air atmosphere for 1 hour; and (3) repeating steps (1) and (2) for 5 times to obtain a fixed bed catalyst used for coupling conversion of naphtha and CO₂ to produce benzene, toluene and p-xylene, named as FXNCC-14, where the amount of the tetraethyl silicate introduced for 6 times was equivalent to the amount introduced for one time in Example 10.

Then, the temperature was adjusted to a reaction temperature of 550°C in a nitrogen atmosphere. A raw material naphtha was fed by a micro-feed pump, and the flow of CO₂ was controlled by a mass flow meter. The mass ratio of the raw material CO₂ to the naphtha was 0.8:1, the weight hourly space velocity of the naphtha was 1.0 h⁻¹, and the reaction pressure was 0.1 MPa. Reaction products were analyzed by on-line Agilent7890 gas chromatography, and sampling was carried out for analysis when a reaction was carried out for 30 minutes. Reaction results are shown in Table 18.

**Table 18 Evaluation of reaction performance of a catalyst in Example 29**

| | | |
|---|---|---|
| Conversion rate of naphtha (wt%) | | 86.61 |
| Conversion rate of CO₂ (wt%) | | 34.63 |
| Selectivity of ethylene and propylene in hydrocarbon products (wt%) | | 8.04 |
| Selectivity of benzene, toluene and PX in hydrocarbon products (wt%) | | 75.08 |
| Selectivity of aromatic hydrocarbons in hydrocarbon products (wt%) | | 78.62 |
| Selectivity of PX in xylene products | | 97.83 |

| Composition of hydrocarbon products (wt%) | | |
|---|---|---|
| | Methane | 2.51 |
| | Ethylene | 3.95 |
| | Ethane | 3.68 |
| | Propylene | 4.09 |
| | Propane | 4.09 |
| | C₄ | 3.06 |
| | Benzene | 20.88 |
| | Toluene | 38.87 |
| | Ethylbenzene | 0.69 |
| | P-xylene | 15.33 |
| | M-xylene | 0.23 |
| | O-xylene | 0.11 |
| | C₈₊ aromatic hydrocarbons | 2.51 |

In addition to the naphtha used in the above examples, any naphtha selected from hydrocracked naphtha, catalytic cracked naphtha, raffinate oil and topped oil or any mixture thereof may also be used in the present application.

The above descriptions are only several examples of the present application, and are not intended to limit the present application in any way. Although the present application is disclosed above through preferred examples, the examples are not intended to limit the present application. For any skilled person familiar with the art, various changes or modifications made by using the technical contents disclosed above without departing from the scope of technical schemes of the present application are considered as equivalent examples, which fall within the scope of the technical schemes.

## Claims

1. A method for preparing a modified molecular sieve catalyst used for catalyzing coupling conversion of naphtha and CO₂ to produce benzene, toluene and p-xylene, wherein the method comprises subjecting a molecular sieve to metal modification by using a high temperature hydrothermal method, comprising the following steps:
(1) preparing a soluble metal salt aqueous solution;
(2) placing a zeolite molecular sieve to be metal-modified in the soluble metal salt aqueous solution, and impregnating the same at a temperature of 60-100°C; and
(3) draining the molecular sieve obtained in step (2), followed by drying and calcination to obtain the modified molecular sieve catalyst.

2. The method according to claim 1, wherein the solid-liquid ratio of the zeolite molecular sieve to be metal-modified to the soluble metal salt aqueous solution is 1/10 to 1/1, and the mass concentration of a metal salt in the soluble metal salt aqueous solution is 10%-30%; the impregnating time is 2-10 hours; the step of drying is carried out in an air atmosphere at 100-150°C; and the step of calcination is carried out in an air atmosphere at 500-700°C.

3. The method according to claim 1, wherein a metal used for the metal modification is selected from at least one of La, Zn, Ga, Fe, Mo and Cr metals.

4. The method according to claim 1, wherein the modified zeolite molecular sieve catalyst consists of a modified HZSM-5 zeolite molecular sieve.

5. The method according to claim 1, wherein the modified zeolite molecular sieve catalyst comprises a modified HZSM-5 zeolite molecular sieve and a binder.

6. The method according to claim 1, wherein the method further comprises subjecting the molecular sieve to silanization modification after the metal modification.

7. The method according to claim 6, wherein the silanization modification is carried out by using an in situ chemical vapor deposition method, and comprises the following steps:
(4) placing the metal-modified zeolite molecular sieve in a reactor;
(5) introducing a material A containing a silanization reagent into the reactor at one time, wherein the introduced amount of the silanization reagent is 0.2-0.3 g/g solid, and the silanization reagent is gaseous in the reactor; and
(6) stopping the introduction of the material A into the reactor, raising the temperature of the reactor to 400°C or above, and introducing air for calcination; wherein preferably, the temperature of the reactor is raised to 400-550°C, and the air is introduced for calcination.

8. The method according to claim 6, wherein the silanization modification is carried out by using an in situ vapor atomic layer deposition method, and comprises the following steps:
(4') placing the metal-modified zeolite molecular sieve in a reactor;
(5') introducing a material A containing a silanization reagent into the reactor at n times, wherein the introduced amount of the silanization reagent each time is 0.03-0.06 g/g solid, the silanization reagent is gaseous in the reactor, and the n is in a value range of 3-6; and
(6') stopping the introduction of the material A into the reactor, raising the temperature of the reactor to 400°C or above, and introducing air for calcination; wherein preferably, the temperature of the reactor is raised to 400-550°C, and the air is introduced for calcination.

9. The method according to claim 6, wherein the silanization reagent used for the silanization modification is selected from at least one of compounds with the following chemical formula: 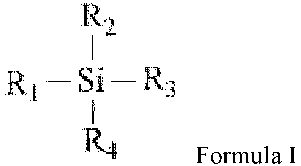 wherein R₁, R₂, R₃ and R₄ are independently selected from C₁₋₁₀ alkyl and C₁₋₁₀ alkoxyl.

10. The method according to claim 9, wherein according to the silanization reagent used for the silanization modification, at least one of the R₁, the R₂, the R₃ and the R₄ is selected from C₁₋₁₀ alkoxyl.

11. The method according to claim 9, wherein the silanization reagent is selected from at least one of tetraethyl silicate and tetramethyl silicate.

12. A method for producing benzene, toluene and p-xylene by coupling conversion of naphtha and CO₂, wherein the method comprises the following steps:
(a) preparing a modified molecular sieve catalyst by the method according to any one of claims 1 to 11; and
(b) enabling a raw material containing naphtha and CO₂ to contact with the modified molecular sieve catalyst in a reactor for a reaction to produce benzene, toluene and p-xylene.

13. The method according to claim 12, wherein the raw material consists of naphtha and CO₂.

14. The method according to claim 12, wherein the naphtha is selected from at least one of hydrocracked naphtha, catalytic cracked naphtha, raffinate oil, topped oil and direct coal liquefied naphtha;
preferably, the carbon number distribution of hydrocarbons in the naphtha is in a range of C₄-C₁₂;
and preferably, the reactor is one of a fixed bed reactor, a fluidized bed reactor or a moving bed reactor.

15. The method according to claim 12, wherein conditions for the reaction of the naphtha and the CO₂ are as follows: the reaction temperature is 450-650°C, the reaction pressure is 0.1-3 MPa, the weight hourly space velocity of the naphtha is 0.1-5 h⁻¹, and the weight hourly space velocity of the CO₂ is 0.1-5 h⁻¹.
